(19) Europäisches Patentamt
European Patent Office
Office européen des brevets

(11) Publication number: **0 115 142**
**B1**

(12) **EUROPEAN PATENT SPECIFICATION**

(45) Date of publication of patent specification: **05.11.86**

(21) Application number: **83307599.7**

(22) Date of filing: **14.12.83**

(51) Int. Cl.$^4$: **C 07 D 405/04,**
C 07 D 311/66, A 61 K 31/415
// (C07D405/04, 311:66, 233:22)

(54) **Chroman compounds.**

(30) Priority: **23.12.82 GB 8236602**

(43) Date of publication of application:
**08.08.84 Bulletin 84/32**

(45) Publication of the grant of the patent:
**05.11.86 Bulletin 86/45**

(84) Designated Contracting States:
**AT BE CH DE FR GB IT LI LU NL SE**

(56) References cited:
**EP-A-0 015 562**
**EP-A-0 033 655**
**DE-A-2 105 191**
**DE-A-2 364 165**
**DE-A-2 413 849**
**FR-A-2 436 785**
**US-A-3 691 187**
**US-A-3 933 847**

(73) Proprietor: **ICI AMERICAS INC**
**Concord Pike & New Murphy Road**
**Wilmington Delaware 19897 (US)**

(72) Inventor: **Ohnmacht, Cyrus John, Jr.**
**2212 Beaumont Road**
**Wilmington Delaware 19803 (US)**

(74) Representative: **Allerton, Roy et al**
**Imperial Chemical Industries PLC Legal Department: Patents PO Box 6**
**Welwyn Garden City Herts, AL7 1HD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention comprises chroman compounds which are useful as anti-depressants as well as in other disease states which are alleviated by blockage of alpha$_2$-adrenoreceptors ($\alpha_2$-adrenoreceptors), pharmaceutical compositions containing such compounds, processes for their synthesis, and novel intermediates used in such syntheses.

The specificatiomn of United States patent No. 2,979,511 purports to disclose compounds of the following formula (A):

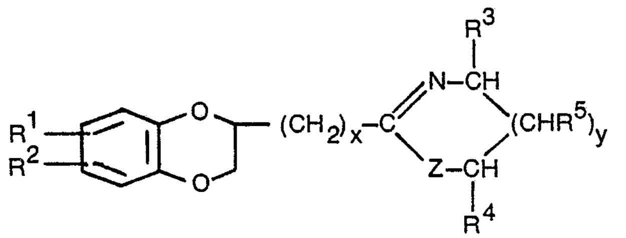

(A)

and acid-addition salts of those compounds which contain a basic nitrogen atom, wherein $R^1$ and $R^2$ are the same or different and represent hydrogen, hydroxy, halogen, trifluoromethyl, nitro, lower alkyl, lower alkenyl or lower alkoxy, $R^3$, $R^4$ and $R^5$ are the same or different and represent hydrogen or lower alkyl, x is zero or one, y is zero or one, and Z is —NH— or —N(lower alkyl)-. The compounds are said to be peripheral vasodilators. Amongst the compounds purportedly described in that specification is 2-[2-(1,4-benzodioxanyl)]-2-imidazoline hydrochloride (Example 1 in said specification), which has the following formula (B):

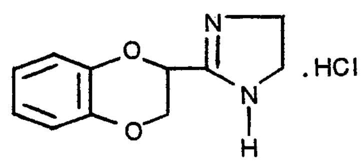

(B)

[The compound of formula (B) is referred hereinafter as RX 781094].

However, it is stated in the specification in European patent application No. 81300427.2 (Publication No. 33655) that that compound is not in fact obtained by the method described in the said Example 1 of U.S. patent No. 2,979,511, and that the compound actually obtained by that method is 2-methyl-2-[2-(1,3-benzodioxolyl)]-2-imidazoline hydrochloride, which has the following formula (C):

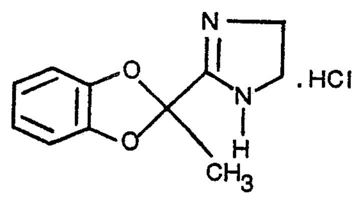

(C)

The said European patent application describes and claims 2-[2-(1,4-benzodioxanyl)]-2-imidazoline or a non-toxic salt thereof, substantially free of 2-methyl-2-[2-(1,3-benzodioxolyl)]-2-imidazoline or a non-toxic salt thereof, and processes for the preparation thereof. It is stated in the said European patent application that the benzodioxanyl derivatives in question have a high degree of selectivity in blocking presynaptic alpha$_2$-adrenoreceptors.

According to the present invention there are provided chroman compounds of the following formula (I):

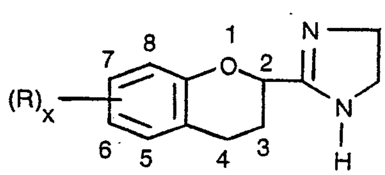

(I)

wherein R is a halogen attached to the 5- and/or 8-position of the molecule; and x is 0, 1 or 2; and the pharmaceutically-acceptable acid-addition salts thereof.

Formula (I) compounds may be described as 2-imidazolin-2-yl chromans.

Particular values for R include fluoro, chloro, bromo and iodo, especially fluoro.

Preferred values of x are 0 and 1, and it is to be understood that when x is 2, the halogens may be the same or different.

When a single halogen substituent R is present, it is at the 5- or 8-position, preferably at the 5-position. When two halogen substituents are present, they are at the 5- and 8-positions.

Particular acid-addition salts include non-toxic pharmaceutically-acceptable salts formed by reaction of the free base with inorganic acids such as hydrochloric, sulfuric or phosphoric and organic acids such as lower alkylcarboxylic acids or diacids such as acetic, propionic, malonic, succinic, fumaric, tartaric, citric or cinnamic.

Due to the presence of the asymmetric carbon atom at the 2-position of the chroman nucleus, the compounds of the formula (I) may exist in the form of optical isomers. It is to be understood that the present invention includes all such optical isomers which exhibit the above-mentioned biological activity.

The compounds of formula (I) may be prepared from the corresponding chroman-2-carboxylic acid of the following formula (II):

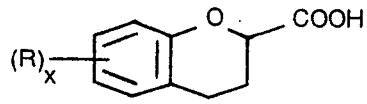

(II)

The chroman 2-carboxylic acid (II) is converted to the corresponding chroman-2-carboxamide (III) by methods known in the art to convert carboxylic acids to amides, e.g., by first converting the acid to the acid chloride and reaction with ammonia, or by first preparing an activated species for example by treatment of the acid with 1,1′-carbonyldiimidazole and reaction of the activated species with ammonia. The resulting chroman-2-carboxamide has the following formula (III):

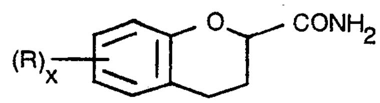

(III)

Reaction of the amide (III) with a dehydrating agent such as phosphorus oxychloride yields the corresponding chroman-2-carbonitrile of the following formula (IV):

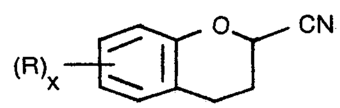

(IV)

Reaction of the nitrile (IV) with ethylenediamine yields the corresponding compound of the formula (I).

Alternatively, the nitrile may be converted to the corresponding imidate ester function of the formula —C(=NH)O-alkyl by reactions corresponding to those set forth in United Kingdom Published Patent Application 2,068,376, followed by reaction with ethylenediamine. The product of the reaction is an imidazoline compound of the formula (I).

In more detail, the acid (II) may be prepared by either the Phenoxylactone Route described by D. T. Witiak et al. in the Journal of Medicinal Chemistry, Vol. 14, No. 8, pages 758—766 (1971) or by the Acetophenone Route as described by V. A. Zagorevskii et al. in Chemical Abstracts, Vol. 55, 22301f (1961) for the chromone-2-carboxylic acid and by J. Augstein et al. in the Journal of Medicinal Chemistry, Vol. 11, pages 844—848 (1968) for the chroman-2-carboxylic acid as summarized below:

Phenoxylactone Route

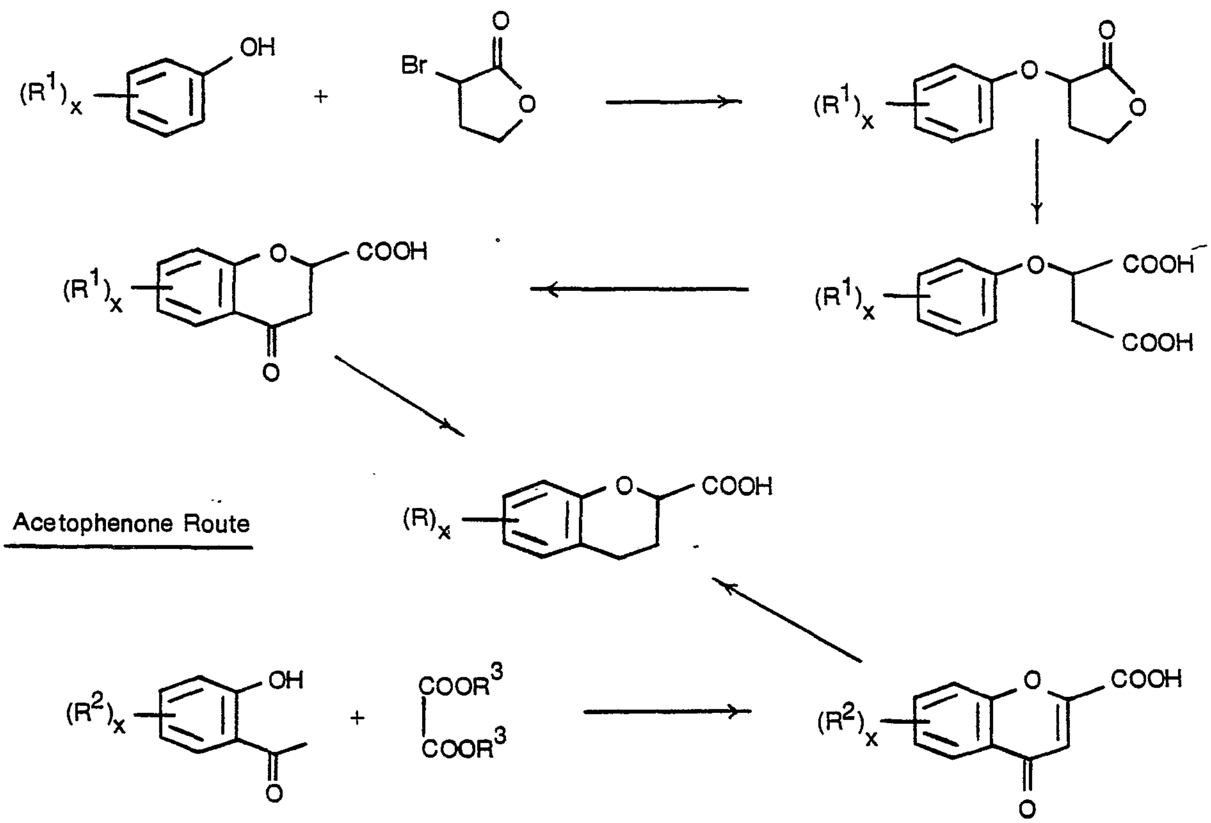

Acetophenone Route

In the above routes, x is as defined for formula (I); $R^1$ is any of the definitions of R; $R^2$ is fluorine attached to the 5- and/or 8- position of the molecule (since the catalytic hydrogenation of the chromone tends to also remove chlorine, bromine or iodine); and $R^3$ is alkyl, e.g., lower alkyl such as methyl or ethyl. In the phenoxylactone route, meta placement of an $R^1$ group on the starting phenol will produce 2 isomers, i.e., the 5- and 7-substituted chroman, and such isomers may be readily separated by techniques such as crystallization or high pressure liquid chromatography. The phenoxylactone route should be used when 5,8-difluoro-2-(2-imidazolin-2-yl)chroman (the compound of formula I where R=5 and 8-fluoro and x=2) is desired.

The chroman-2-carboxylic acid (II) with the desired R substitution on the benzene ring may be converted to the corresponding chroman-2-carboxamide (III), the unsubstituted chroman being described by J. Augstein et al. in United Kingdom Patent 1,004,468, August 21, 1963 and in Chemical Abstracts *63*:18036e (1965), by reaction with a source of —$NH_2$, e.g., directly with ammonia at elevated temperature or through an activated species such as the acid chloride or by reaction with 1,1′-carbonyldiimidazole which may be obtained from Aldrich Chemical Co. of Milwaukee, Wisconsin, as described above. Chroman-2-carbonitrile (IV) may be obtained from the carboxamide (III) by reaction with a dehydrating agent such as phosphorus pentoxide, phosphorus pentachloride, thionyl chloride or phosphorus oxychloride, neat or in an aprotic solvent such as tetrachloroethane or toluene, at a temperature of about 80 to 120°C. Reaction of the nitrile (IV) with ethylenediamine with a catalyst such as hydrogen sulfide or carbon disulfide, the use of such catalysts being described by R. J. Ferm et al. in Chemical Reviews, Vol. 54, pages 593—613 (1954) and by L. H. Werner et al. in the Journal of Medicinal Chemistry, Vol. 8, pages 74—80 (1965), neat or in a solvent such as toluene, preferably neat, at a temperature of 60 to 120°C yields the desired 2-(2-imidazolin-2-yl)-chroman (I).

According to a further feature of the invention there is provided a process for preparing the compounds of the formula (I), wherein R and x have the meanings stated above, and pharmaceutically-acceptable acid-addition salts thereof, which comprises reacting a compound of the following formula (IV):

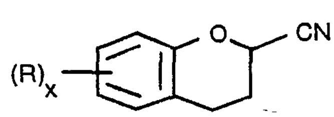

(IV)

wherein R and x have the meanings stated above, with ethylenediamine.

This process is preferably carried out at a moderately elevated temperature, for example at 60° to 120°C, in the presence of a catalytic amount of hydrogen sulfide or carbon disulfide. Also, the process is conveniently carried out in an inert atmosphere, for example under nitrogen.

The role of alpha$_2$-adrenoreceptors in the disease states such as depression, cardiac failure and excessive bronchoconstriction, e.g., to alleviate asthma and hay fever in humans, has been described in European Published Patent Application No. 33,655 issued August 22, 1981, in particular the compound RX 781094 described therein, and by D. S. Charney in Arch. Gen. Psychiatry, Vol. 38, pages 1160—1180 (1981). The compounds of the present invention are potent alpha$_2$-antagonists and are thus useful in the treatment of disease states such as depression as set forth by P. Timmermans et al. in the Journal of Medicinal Chemistry, Vol. 25, No. 12, pages 1389—1401 (1982) and as evidenced by tests which are described below.

Measurement of alpha$_2$-antagonism, and thus of utility as an antidepressive pharmaceutical, is shown in the following tests.

*Tritiated Yohimbine Binding Assay:* This test measures the affinity of alpha$_2$-agonists and alpha$_2$-antagonists for the alpha$_2$ binding site in human platelets, which is believed to be similar to the alpha$_2$ binding site in the human brain. The test does not rigorously distinguish between alpha$_2$-agonists and alpha$_2$-antagonists but provides a measure of the intrinsic potency of such compounds at the alpha$_2$ binding site. Compounds found active are subsequently categorised as alpha$_2$-agonists or alpha$_2$-antagonists on the basis of their activities in the Audiogenic Seizure, Clonidine Hypoactivity and Locus Coeruleus Tests described below. The test is conducted as described by M. Daiguji et al. in Life Sciences, Vol. 28, pages 2705—2717 (1981). Platelets are washed and disrupted as described in the Daiguji article. The amount of platelet used per assay varies between 0.2 to 1.5 mg of protein. The binding assay consists of 1 ml of 50 millimolar Tris hydrochloride (pH=7.0 at 25°C), 1 ml of platelet preparation and 20 microliters of 2 $\times 10^{-10}$ molar tritiated yohimbine. For the determination of non-specific binding, 20 microliters of 10 millimolar norepinephrine is used. The incubation is conducted at 25°C for 60 minutes, after which the membranes are filtered under vacuum on a GF/B filter. The filters are then counted in a scintillation counter. The $IC_{50}$ values for compounds (I) of this invention were: the compound of Example 1, 34 nanomolar; the compound of Example 2e, 3.8 nanomolar; the compound of Example 3e, 53.5 nanomolar. The $IC_{50}$ for yohimbine, a reference agent and a known alpha$_2$-antagonist, is 2 nanomolar. $IC_{50}$ is the concentration of test compound that will cause a 50% reduction of the specific binding for $^3$H-yohimbine. It is known that if an alpha$_2$-agonist or alpha$_2$-antagonist binds to alpha$_2$ sites, such will be displaced more easily by a compound which is also of the same type, i.e., an agonist or antagonist, respectively.

*$^3$H-Clonidine Binding Assay.* On a tritiated clonidine binding assay, as described by A. Salama et al. in Life Sciences, Vol. 30, pages 1305—1311 (1982), the $IC_{50}$ of the compound of the present invention produced in Example 1c is 110 nanomolar which indicates that the compound is an alpha$_2$-antagonist rather than an alpha$_2$-agonist.

*Audiogenic Seizure (AS):* Sound-produced seizures in mice are blocked by clonidine, an alpha$_2$-agonist, and such a model may be used to measure the activity of a particular compound as an alpha$_2$-antagonist, specifically as it decreases clonidine activity. The activity of clonidine in this regard and its relationship to alpha$_2$-receptors is described by R. Horton et al. in the Journal of Pharmacology and Experimental Therapeutics, Vol. 214, No. 2, pages 437—442 (1980). DBA—2 mice (20—22 days of age; 8 to 12 g) are used in these studies. Mice are tested individually in a glass bell jar (11½ inches high and 12 inches in diameter) which is placed in a sound-attenuated chamber. An electric bell is used to generate a noise level of 116—118 decibels. Mice are allowed to acclimate to the chamber for 30 seconds and are then exposed to the sound for 60 seconds. The auditory stimulation causes a convulsive episode characterized by wild running, clonus, tonus and death. Clonidine (0.4 mg/kg of body weight, i.p.), an alpha$_2$-noradrenergic agonist, injected 30 minutes prior to auditory stimulation, will protect 100% of the mice from audiogenic seizures. The protective effect of the alpha$_2$-agonist can be reversed by pretreatment with agents that are preferentially alpha$_2$-antagonists, e.g., yohimbine. Thus, the test compound is dissolved in a hydroxypropylmethylcellulose (HPMC) vehicle consisting of 0.1% Tween® 80 and 0.5% hydroxypropylmethylcellulose in an 0.9% by weight sodium chloride aqueous solution and injected intraperitoneally (i.p.) into the mouse. (Tween® is a registered trademark of ICI Americas Inc., Wilmington, Delaware, USA, for emulsifiers, wetting and dispersing agents. Tween® is polyoxyethylene sorbitan mono-oleate). After one hour, the mouse is challenged with clonidine intraperitoneally at 0.4 mg/kg of body weight. After a further 30 minutes, the mouse is subjected to a tone from a high intensity bell and the mouse is observed for seizure activity. The activity is scored on the basis of whether clonic and/or tonic seizures occur. Minimal effective doses (MED) for compounds of the invention, i.e., the compounds of Examples 1c, 2e, and 3e, and for the compound of European Published Patent Application 33,655, i.e., RX 781094, are reported in Table 1 below.

*Clonidine Hypoactivity (CH) Test:* This is an in vivo test to determine the ability of the test compound to antagonize the effect of clonidine, an alpha$_2$-agonist which produces a potent suppression of spontaneous locomotor activity. The suppression can be prevented by administration of an alpha$_2$-antagonist as described by J. Malick et al. in Federation Proceedings, Vol. 40, page 244 (1981), a publication of the Federation of the American Societies of Experimental Biology. Male Sprague-Dawley (HLA) rats, weighing 140 to 180 g, were used throughout these studies. Spontaneous locomotor activity was measured in Animex activity monitors supplied by Columbus Instruments, Columbus, Ohio; groups of three animals

were placed in a chamber of clear "Plexiglas", 26.5 × 26.5 × 15 cm for the test. ("Plexiglas" is a trademark of Rohm & Haas Co., Philadelphia, Pennsylvania, USA, for thermoplastic polymethyl methacrylate type polymers and plastic sheets). All animals received two injections prior to testing according to the following paradigm: vehiclec (HPMC) or antagonist was administered subcutaneously 30 minutes prior to intraperitoneal administration of either HPMC or clonidine. As a result of several pilot studies, 0.01 mg/kg of body weight of clonidine given i.p., was chosen as a standard dose; this was the lowest dose that consistently produced a statistically significant reduction in motor activity. Animals were placed into the motor activity chambers as soon as possible after the second injection and total activity counts were recorded for 20 minutes. Five groups of 3 rats each were used for each dose of drug or control. Results were statistically evaluated by comparing the drug-treated groups to the appropriate same-day vehicle-treated group and the clonidine-treated group using the Student's t-test. Minimal effective dosages for the compounds of the invention produced in Examples 1c, 2e and 3e, and for compound RX 781094 (all in mg/kg of body weight, subcutaneously) are given in Table 1 below.

*Mouse Learned Helplessness Swimming test* (MLHS): The MLHS test is predictive of antidepressant activity in a compound as described by R. D. Porsolt in the Chapter "Behavioral Despair" in "Antidepressants: Neurochemical, Behavioral, and Clinical Perspectives", Edited by S. J. Enna et al., pages 121—139, Raven Press, New York (1981). In the one-trial mouse learned helplessness swimming test, drugs are typically administered intraperitoneally one hour before exposing the mouse to an inescapable situation, i.e., a "Plexiglas" jar half-filled with water. During testing, a mouse is gently placed in the water and tested for a total of 6 minutes as follows: during the first 2 minutes the animal quickly "learns" there is no escape and spends the vast majority of the last 4 minutes floating (tonic immobility). Nothing is recorded during the first 2 minutes (learning period) but the time in seconds spent in tonic immobility during the last 4 minutes of the session is recorded. Typically, controls average between 200—220 seconds of the total 240 seconds in tonic immobility. Control (vehicle-treated) groups are run each day and drug-treated groups are compared to these via a Student's t-test. Effective anti-depressants will significantly reduce the time spent in tonic immobility. Minimal effective doses (mg/kg of body weight i.p.) for compounds of this invention (Examples 1c, 2e and 3e) and for imipramine, a known anti-depressant, are given in Table 1 below.

TABLE 1

Minimal effective doses (MED), mg/kg of body weight, in audiogenic seizure (AS), clonidine hyperactivity (CH), and mouse learned helplessness swimming (MLHS) tests

| Compound | Test AS (i.p.) | CH (s.c.) | MLHS (i.p.) |
|---|---|---|---|
| Test compounds: | | | |
| Example 1c | 5 | 0.3 | 10 |
| Example 2e | 1 | 0.5 | 30 |
| Example 3e | 3* | IA | 30 |
| Comparison compounds: | | | |
| RX 781094 | 2.5 | 0.03 | IA |
| Imipramine | IA | IA | 7.5 |

*Approximate
IA denotes essentially inactive in this test
i.p. denotes intraperitoneal administration
s.c. denotes subcutaneous administration

All three test compounds showed some alpha$_2$antagonist activity. The most active compounds, as shown by the test results taken collectively, were 2-(2-imidazolin-2-yl)-5-fluorochroman (the compound of Example 2e) and 2-(2-imidazolin-2-yl)chroman (the compound of Example 1c) in the order named.

*Locus Coeruleus Test:* The locus coeruleus test for alpha$_2$-antagonist activity is predictive of anti-depressant activity as described by G. Engberg et al. in Communications in Psychopharmacology, Vol. 4, pages 233—239 (1980). The alpha$_2$-antagonistic properties of a test agent are determined as follows: Male Sprague-Dawley rats (280—320 g) are anesthetized with sodium pentobarbitol (60 mg/kg of body weight, i.p.) and placed in a stereotaxic instrument to allow precise location of the electrode in the locus coeruleus. Extracellular single unit activity is recorded from the locus coeruleus using stainless steel or tungsten microelectrodes (2—5 megohm impedance). Extracellular potentials are counted during successive 10 second intervals and recorded on a digital counter. The baseline firing rate of each neuron is determined for 3 to 5 minutes before drug administration. Drugs are injected intravenously in a volume of 0.1 ml; data are

collected for 2 to 3 minutes between multiple drug injections. The ability of a test agent to reverse the suppressant effects of clonidine, and the total cumulative dose of clonidine required to override the test agent, are indicative of alpha$_2$-antagonistic properties. In this test, 10 micrograms/kg of body weight of clonidine were administered by infusion intravenously to suppress firing. Yohimbine, an alpha$_2$-antagonist, reinstated firing when infused at 2.0 mg/kg of body weight i.v. Subsequently, 160 micrograms/kg of body weight of clonidine were required to re-suppress firing. In contrast, the compounds of Examples 1c and 2e reinstated firing at the same level, but much larger amounts of clonidine, i.e., 1280 micrograms/kg of body weight (compound of Example 1c), and 2560 mg/kg (compound of Example 2e), were required to re-suppress firing.

None of the compounds of the formula (I) which have been tested in the above-mentioned tests has exhibited any signs of toxicity in the mouse at dosages up to 30 mg/kg administered intraperitoneally.

According to a further feature of the invention there are provided pharmaceutical compositions comprising at least one compound of the formula (I), wherein R and x have the meanings stated above, or a pharmaceutically-acceptable acid-addition salt thereof, and a pharmaceutically-acceptable diluent or carrier.

Compounds of the present invention may be administered in the form of an oral, rectal or parenteral dosage, e.g., as a tablet, capsule, solution or suspension, by compounding methods known in the pharmaceutical art. The compounds may be used singly, in combination with each other, in combination with another active ingredient, such as a tricyclic antidepressant, for example imipramine, or with an anti-anxiety agent, e.g., diazepam. For depression, compounds of the invention would be given at an oral dosage of about 5 to 30 mg/kg of body weight per day in single or multiple administration. Thus, for an average human in need of treatment for depression, about 300 to 1800 mg per day of a compound of the present invention would be administered orally, e.g., divided into 4 equal doses. If the compound is to be given intramuscularly, the dosage would be about 5 to 30 mg/kg of body weight per day, e.g., divided into 4 equal doses.

According to a further feature of the invention there are provided, as novel compounds which are useful as intermediates, the compounds of the following formula (IV):

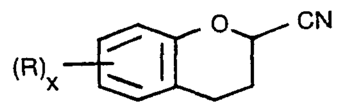

(IV)

wherein R and x have the meanings stated above.

The following Examples illustrate the preparation of compounds (I) of this invention and intermediates. In the following Examples and throughout the specification, the following abbreviations are used: mg (milligrams); g (grams); °C (degrees Centigrade); kg (kilograms); ml (milliliters); mp (melting point); i.p. (intraperitoneal); cm (centimeters); i.v. (intravenously); and C, H, N, O, etc. (the conventional symbols for the elements).

Example 1

a. *Chroman-2-carboxamide* (Formula III; x=0)

(1) To 5.00 g (0.0281 mole) of chroman-2-carboxylic acid and 4.55 g (0.0281 mole) of 1,1'-carbonyldiimidazole was added 100 ml of dry tetrahydrofuran and the solution was stirred at reflux under nitrogen for 1 hour. The mixture was then cooled to room temperature and gaseous ammonia was bubbled through the stirred solution for 0.5 hour. The solvent was removed in vacuo and the residue treated with water. The resulting white solid weighed 3.56 g (71% yield), mp 126.5 to 127.5°C (literature mp 125—6°C, J. Augstein, A. M. Monro and T. I. Wrigley British Patent 1,004,468 (August 21, 1963) to Pfizer Ltd.; Chemical Abstracts 63:18036e (1965)).

2. To a solution of 36.92 g (0.228 mole) of 1,1'-carbonyldiimidazole in 800 ml of dry tetrahydrofuran was added portionwise 40.57 g (0.228 mole) of chroman-2-carboxylic acid and the mixture was stirred at reflux under nitrogen for 1 hour. The reaction mixture was then cooled (ice-bath) and gaseous ammonia bubbled through the solution for 25 minutes. The solvent was removed in vacuo and the residue triturated with 400 ml of water. The resulting white solid weighed 33.70 g (84% yield), mp 125.5—127.5°C (literature mp 125—6°C, J. Augstein et al, see above).

b. *Chroman-2-carbonitrile* (Formula IV; x=0)

(1) A stirred solution of 3.38 g (0.0191 mole) chroman-2-carboxamide in 65 ml of dry toluene was treated with 8.77 g (0.0572 mole) of phosphorus oxychloride and the mixture heated at reflux for 0.5 hour. The cooled reaction mixture was treated with ice and water, the layers separated and the aqueous phase extracted with three 200 ml portions of ethyl acetate. The combined organic phase was washed with saturated sodium chloride solution and dried over $MgSO_4$. The solution was filtered and evaporated to yield a green oil. Kugelrohr distillation at 85°C (bath temperature) (0.005 torr) returned 2.83 g (93%) of yellow oil.

| | | | |
|---|---|---|---|
| Anal. Cal. for $C_{10}H_9NO$ (159.19): | C, 75.45; | H, 5.70; | N, 8.80 |
| Found: | C, 74.11; | H, 5.72; | N, 8.51 |

(2) A stirred slurry of 33.60 g (0.19 mole) of chroman-2-carboxamide in 650 ml of dry toluene was treated with 87.2 g (0.57 mole) of phosphorus oxychloride and the mixture heated at reflux for 0.5 hour. The cooled reaction mixture was treated with ice and water, the layers separated and the aqueous phase extracted with four 700 ml portions of ethyl acetate. The combined organic phase was washed with saturated sodium chloride solution and dried over $MgSO_4$. The solution was filtered and evaporated to yield a black oil. Kugelrohr distillation at 110—130°C (bath temperature) (0.25 torr) gave 26.23 g of colorless oil which was chromatographed by the flash chromatography method, see W. C. Still et al. in the Journal of Organic Chemistry, Vol. 43, page 2923 (1978), on a 7" × 2½" diameter column of silica gel (E. Merck No. 9385, 400—230 mesh) using toluene as eluant. Evaporation of the appropriate fractions returned 10.23 g (34% yield) pure chroman-2-carbonitrile as a colorless oil.

| | | | |
|---|---|---|---|
| Anal. Cal. for $C_{10}H_9NO$ (159.19): | C, 75.45; | H, 5.70; | N, 8.80 |
| Found: | C, 75.22; | H, 5.92; | N, 9.01 |

c. *2-(2-Imidazolin-2-yl)chroman* (Formula I; x=0)

(1) A stirred mixture of 1.30 g (0.0082 mole) of chroman-2-carbonitrile, 0.99 g (0.0164 mole) of ethylenediamine and 2 drops of carbon disulfide was heated at 90°C under $N_2$ for 18 hours. The resulting yellow oil was combined with reaction product from an earlier run (0.20 g (0.0013 mole) of chroman-2-carbonitrile) and the total chromatographed by the flash chromatography method on a 7" × 1" diameter column of silica gel (E. Merck No. 9385, 400—230 mesh) using 20% methanol in chloroform as eluant. Evaporation of the appropriate fractions returned 1.32 g of pale yellow solid which was dissolved in warm absolute ethanol and treated with excess ethereal hydrogen chloride. The resulting white hydrochloric salt of 2-(2-imidazolin-2-yl)chroman weighed 1.22 g (54% yield), mp 255—7°C.

| | | | | |
|---|---|---|---|---|
| Anal. Cal. for $C_{12}H_{14}N_2O.HCl$ (238.72): | C, 60.38; | H, 6.33; | N, 11.74; | Cl, 14.85 |
| Found: | C, 60.44; | H, 6.43; | N, 11.65; | Cl, 15.18. |

(2) A stirred mixture of 7.87 g (0.049 mole) of chroman-2-carbonitrile, 5.89 g (0.098 mole) of ethylenediamine and 5 drops of carbon disulfide was heated at 70°C under nitrogen for 18 hours. The resulting yellow oil was treated with a total of 250 ml refluxing hexane and the clear hexane solution decanted from a small amount of insoluble viscous yellow oil. Four crops of solid were taken from the hexane solution; 7.10 g, mp 88.5—90.5°C, 1.37 g, mp 86.5—88.5, 0.62 g, mp 85—88°C and 0.07 g, mp 83.5—85°C. Total yield was 9.16 g (92% yield) of white solid.

| | | | |
|---|---|---|---|
| Anal. Cal. for $C_{12}H_{14}N_2O$ (202.26): | C, 71.26; | H, 6.98; | N, 13.85 |
| Found: | C, 71.09; | H, 6.83; | N, 13.79 |

Treatment of an ethereal solution of 8.10 g (0.04 mole) of the above free base with ethereal hydrogen chloride returned 9.20 g (96% yield) of white hydrochloride salt, mp 260.5—262.5°C.

| | | | | |
|---|---|---|---|---|
| Anal. Cal. for $C_{12}H_{14}N_2O.HCl$ (238.72): | C, 60.38; | H, 6.33; | N, 11.74; | Cl, 14.85 |
| Found: | C, 60.11; | H, 6.37; | N, 11.78; | Cl, 14.73 |

Example 2

a. *5-Fluoro-4-oxo-4H-1-benzopyran-2-carboxylic acid*

To a stirred solution of sodium ethoxide in ethanol (from 7.88 g (0.343 mole) of sodium and 207 ml of absolute ethanol), under a nitrogen atmosphere, was added a solution of 12.19 g (0.079 mole) of 2-fluoro-6-hydroxyacetophenone and 25.43 g (0.174 mole) of diethyl oxalate at such a rate as to initiate and maintain a mild reflux. The solution was refluxed for an additional 0.5 hour and the solvent distilled off *in vacuo* to yield a yellow solid. The solid was treated with an excess of a solution of 6% acetic acid in water. The solvent was removed *in vacuo* and the residual yellow solid dissolved in 200 ml of glacial acetic acid, treated with 50 ml of 37% hydrochloric acid and stirred and heated at 80° for 2 hours. After removal of the solvent *in vacuo,* the brown residue was triturated with water and the solid filtered off. The yield was 5.11 g (31%) of material with mp 240—7° dec. A sample from an analogous run, recrystallized from acetonitrile, had mp 239—242.5° (dec).

| | | |
|---|---|---|
| Anal. Cal. for $C_{10}H_5FO_4.\frac{1}{4}H_2O$ (212.65): | C, 56.48; | H, 2.49 |
| Found: | C, 56.19; | H, 2.81 |

b. *5-Fluorochroman-2-carboxylic acid* (Formula II; R=5—f, x=1)

A mixture of 5.01 g (0.024 mole) of 5-fluoro-4-oxo-4H-1-benzopyran-2-carboxylic acid, 0.5 g of 10% Pd-C and 95 ml of glacial acetic acid was hydrogenated on a Parr apparatus at 50 psig and 50° for 18 hours. The catalyst was filtered off through a "Celite" diatomaceous earth pad and the acetic acid removed *in vacuo.*

The residue, recrystallized from hexane, returned 3.41 g (72%) of product, mp 105—112°.

| | | |
|---|---|---|
| Anal. Cal. for $C_{10}H_9FO_3$ (196.18): | C, 61.23; | H, 4.62 |
| Found: | C, 61.34; | H, 4.89 |

c. *5-Fluorochroman-2-carboxamide* (Formula III; R=5—F, x=1)

To a mixture of 3.37 g (0.017 mole) of 5-fluorochroman-2-carboxylic acid and 2.79 g (0.017 mole) of 1,1′-carbonyldiimidazole, under a nitrogen atmosphere, was added 102 ml of dry tetrahydrofuran. Carbon dioxide gas was immediately liberated and the solution was stirred and refluxed for 0.5 hour. The stirred mixture was then cooled in an ice-bath as ammonia gas was bubbled through the solution for 0.5 hour. After standing overnight at room temperature the solvent was distilled off *in vacuo* and the residue triturated with 100 ml of water. The white solid was filtered off and dried *in vacuo* to yield 2.74 g (81%) of amide, mp 161—163°.

| | | | |
|---|---|---|---|
| Anal. Cal for $C_{10}H_{10}FNO_2$ (195.20); | C, 61.53; | H, 5.16; | N, 7.18 |
| Found: | C, 61.28; | H, 5.26; | N, 7.16 |

d. *5-Fluorochroman-2-carbonitrile* (Formula IV; R=5—F, x=1)

A stirred mixture of 2.70 g (0.0138 mole) of 5-fluorochroman-2-carboxamide and 3.9 ml (0.042 mole) of phosphorus oxychloride in 50 ml of toluene, under a nitrogen atmosphere, was stirred at reflux for 1.5 hours. The reaction mixture was cooled and poured onto 100 ml of ice water, the layers separated and the aqueous phase extracted twice with 100 ml portions of ethyl acetate. The combined organic phase was washed with 75 ml of saturated sodium chloride solution, dried ($Na_2SO_4$), filtered and the solvent removed *in vacuo*. The residual dark brown oil was chromatographed by the flash chromatography method on a 7″ × 1¼″ diameter column of silica gel (E. Merck No. 9385, 400—230 mesh) using toluene/hexane (2:1) as eluant to yield 2.36 g (96%) of the nitrile as a clear colorless oil.

| | | | |
|---|---|---|---|
| Anal. Cal. for $C_{10}H_8FNO$ (177.18): | C, 67.79; | H, 4.55; | N, 7.91 |
| Found: | C, 68.01; | H, 4.74; | N, 7.68 |

e. *2-(2-Imidazolin-2-yl)-5-fluorochroman* (Formula I; R=5—F, x=1)

A stirred mixture of 2.29 g (0.0129 mole) of 5-fluorochroman-2-carbonitrile, 1.64 g (0.0273 mole) of ethylenediamine and 2 drops of carbon disulfide, under a nitrogen atmosphere, was heated at 110° for 18 hours. The crude mixture was chromatographed by the flash chromatography method on a 7″ × 1½″ diameter column of silica gel (E. Merck No. 9385, 400—230 mesh) using 95% ethanol as eluant. The solid thus obtained was dissolved in 50 ml of methanol and treated with a solution of hydrogen chloride in ether. The solvent was removed *in vacuo* and the residue recrystallized twice from absolute ethanol-ether to yield 2.47 g (75%) of the hydrochloride salt of 2-(2-imidazolin-2-yl)-5-fluorochroman as a white solid, mp 263.5—270°C (dec).

| | | | | |
|---|---|---|---|---|
| Anal. Cal. for $C_{12}H_{13}FN_2O.HCl$ (256.71): | C, 56.15; | H, 5.50; | N, 10.91; | $Cl^-$, 13.81 |
| Found: | C, 55.92; | H, 5.53; | N, 10.84; | $Cl^-$, 14.05 |

## Example 3

a. *8-Fluoro-4-oxo-1H-1-benzopyran-2-carboxylic acid*

To a stirred solution of sodium ethoxide in ethanol (from 15.95 g (0.69 mole) of sodium and 267 ml of absolute ethanol), under a nitrogen atmosphere, was added a solution of 25.00 g (0.16 mole) of 3-fluoro-2-hydroxyacetophenone and 48 ml (0.35 mole) of diethyl oxalate at such a rate as to initiate and maintain a mild reflux. A yellow solid formed. The mixture was then refluxed for an additional 0.5 hour. The yellow solid was filtered onto a pad of "Celite" diatomaceous earth, dissolved in 3 liters of hot water and filtered from the Celite. The yellow aqueous filtrate was concentrated to 1 liter *in vacuo,* treated with 35 ml of acetic acid, stirred for 2 hours and the yellow solid filtered off. Evaporation of the solvent from the filtrate gave a second crop of yellow solid. The two crops were treated separately with 200 ml of acetic acid and 50 ml of 37% hydrochloric acid, stirred and heated at 80° for 2 hours and treated with 242 ml of water. Solid from the reaction of crop 1 was filtered off and dried to yield 10.81 g of solid. The filtrate was added to the solution from the reaction of crop 2 and the solvent removed *in vacuo* to yield a yellow solid which, after trituration with 300 ml of water, filtration and drying, returned 10.45 g of material. The total crude yield was 21.26 g (64%). A sample after recrystallization from acetic acid and sublimation at 80°/0.005 mm had mp 217—221° (dec).

| | | |
|---|---|---|
| Anal. Cal. for $C_{10}H_5FO_4$ (208.15): | C, 57.71; | H, 2.42 |
| Found: | C, 57.04; | H, 2.60 |

b. *8-Fluorochroman-2-carboxylic acid* (Formula II; R=8—F, x=1)

A mixture of 20.87 g (0.40 mole) of 8-fluoro-4-oxo-4H-1-benzopyran-2-carboxylic acid, 2 g of 10% Pd-C and 385 ml of glacial acetic acid was hydrogenated on a Parr apparatus at 40 psi. The mixture was heated and an exotherm occurred at *ca* 44°; the temperature rising to 50°. The temperature was maintained at 50° for 45 min., then 60° for 16.5 hours. The catalyst was filtered off through a Celite pad and the acetic acid removed *in vacuo* to yield an off-white solid. The solid was triturated with warm hexane, cooled to room temperature and the solid filtered off. The yield of vacuum dried material was 8.93 g (46%), mp 87—90°. An impure second crop of 0.71 g (4%) was obtained on concentration of the liquors. A sample purified by sublimation at 60°/0.005 mm had mp 93—96°.

Anal. Cal. for $C_{10}H_9FO_3$ (196.18): C, 61.23; H, 4.62
Found: C, 61.29; H, 4.79

c. *8-Fluorochroman-2-carboxamide* (Formula III; R=8—F; x=1)

To a mixture of 9.63 g (0.049 mole) of 8-fluorochroman-2-carboxylic acid and 7.95 g (0.049 mole) of 1,1'-carbonyldiimidazole, under a nitrogen atmosphere, was added 170 ml of dry tetrahydrofuran. Carbon dioxide gas was immediately liberated and the solution was stirred and refluxed for 1 hour. The reaction mixture was then cooled in an ice-bath as ammonia gas was bubbled through the solution for 0.5 hour. The solvent was removed *in vacuo* and the residue triturated with 200 ml of water. The dried white solid weighed 7.65 g (80%). A sample recrystallized from ethyl acetate had mp 146.5—148°.

Anal. Cal. for $C_{10}H_{10}FNO_2$ (195.20): C, 61.53; H, 5.16; N, 7.18
Found: C, 61.67; H, 5.61; N, 6.77

d. *8-Fluorochroman-2-carbonitrile* (Formula IV; R=8—F, x=1)

Phosphorus oxychloride (17.65 g, 0.115 mole) was added to a stirred solution of 7.49 g (0.0384 mole) of 8-fluorochroman-2-carboxamide in 130 ml of dry toluene and the mixture refluxed for 45 minutes. The cooled (ice-bath) mixture was then treated with ice, the layers separated and the aqueous phase extracted with three 200 ml portions of ethyl acetate. The combined organic phase was washed with 300 ml of saturated sodium chloride solution, dried ($Na_2SO_4$), filtered and evaporated to a brown oil. Chromatography by the flash chromatography method on a 7" × $1\frac{1}{2}$" diameter column of silica gel (E. Merck No. 9384, 400—230 mesh) using chloroform as eluent returned 6.78 g (99%) of the nitrile as a gold colored oil.

Anal. Cal. for $C_{10}H_8FNO$ (177.18): C, 67.79; H, 4.55; N, 7.91
Found: C, 67.89; H, 4.87; N, 7.79

e. *2-(2-Imidazolin-2-yl)-8-fluorochroman* (Formula I, R=8—F, x=1)

A solution of 2.50 g (0.014 mole) of 8-fluorochroman-2-carbonitrile, 1.70 g (0.028 mole) of ethylenediamine and two drops of carbon disulfide was stirred and heated, under a nitrogen atmosphere, at 110° for 18 hours. Excess ethylenediamine was removed from the yellow solid *in vacuo* (water aspirator, then vacuum pump) and the residue chromatographed by the flash chromatography method on a 7" × $1\frac{1}{4}$" diameter column of silica gel (E. Merck No. 9385, 400—230 mesh) using 95% ethanol as eluant. The yellow solid thus obtained was dissolved in 50 ml of methanol and treated with hydrogen chloride gas for 10 minutes. The solvent was evaporated and the resulting solid triturated with ether and filtered. The solid was dissolved in refluxing methanol, treated hot with activated carbon (Darco®) and filtered through a "Celite" pad. The resulting solid was the hydrochloride salt of 2-(2-imidazolin-2-yl)-8-fluorochroman, yield 1.50 g (42%), mp >295°. (Darco® is the registered trademark of ICI Americas Inc., Wilmington, Delaware, U.S.A. for activated carbon).

Anal. Cal. for $C_{12}H_{13}FN_2O.HCl$ (256.71): C, 56.15; H, 5.50; N, 10.91; Cl, 13.81
Found: C, 56.11; H, 5.65; N, 10.61; Cl, 14.05

**Claims for the Contracting States: BE CH DE FR GB IT LI LU NL SE**

1. A chroman derivative of the following formula (I):

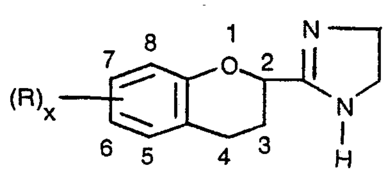

(I)

wherein R is a halogen attached to the 5- and/or 8-position of the molecule; and x is 0, 1 or 2; or a pharmaceutically-acceptable acid-addition salt thereof.

2. A compound as claimed in Claim 1 wherein x is 0 or 1.

3. A compound as claimed in Claim 1 or 2 wherein x is 1 and the halogen substituent R is at the 5-position.

4. A compound as claimed in any one of Claims 1 to 3 wherein R is fluoro.

5. A compound as claimed in Claim 1 which is 2-(2-imidazolin-2-yl)chroman or a pharmaceutically-acceptable acid-addition salt thereof.

6. A compound as claimed in Claim 1 which is 2-(2-imidazolin-2-yl)-5-fluorochroman or a pharmaceutically-acceptable acid-addition salt thereof.

7. A process for preparing a compound of the following formula (I):

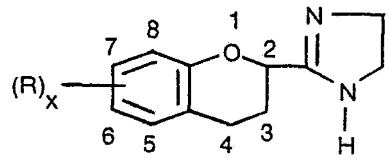

(I)

wherein R and x have the meanings stated in Claim 1, or a pharmaceutically-acceptable acid-addition salt thereof, which comprises reacting a compound of the following formula (IV):

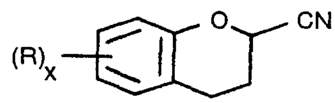

(IV)

with ethylenediamine.

8. A process as claimed in Claim 7 which comprises converting a chroman-2-carboxylic acid of the following formula (II):

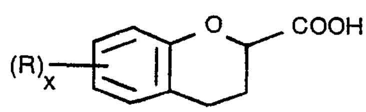

(II)

to the corresponding chroman-2-carboxamide of the following formula (III):

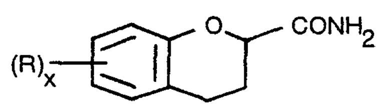

(III)

and reacting the said carboxamide (III) with a dehydrating agent to form said chroman-2-carbonitrile of the following formula (IV):

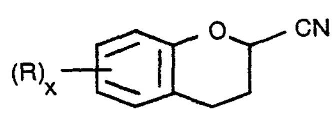

(IV)

and reacting said nitrile (IV) with ethylenediamine to form the corresponding compound of the formula (I), and wherein R and x have the meanings stated in Claim 1.

9. A pharmaceutical composition comprising at least one compound of the formula (I) stated in Claim 1, wherein R and x have the meanings stated in Claim 1, or a pharmaceutically-acceptable acid-addition salt thereof, and a pharmaceutically-acceptable diluent or carrier.

10. A compound of the following formula (IV):

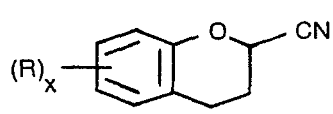

(IV)

wherein R is a halogen attached to the 5- and/or 8-position of the molecule; and x is 0, 1, or 2.

**Claims for the Contracting State: AT**

1. A process for preparing a compound of the following formula (I):

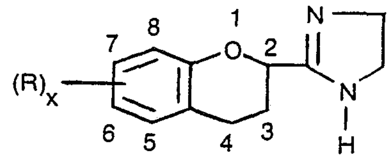

(I)

wherein R is a halogen attached to the 5- and/or 8-position of the molecule, and x is 0, 1 or 2, or a pharmaceutically-acceptable acid-addition salt thereof, which comprises reacting a compound of the following formula (IV):

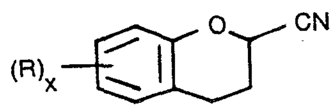

(IV)

with ethylenediamine.

2. A process as claimed in Claim 1 which comprises converting a chroman-2-carboxylic acid of the following formula (II):

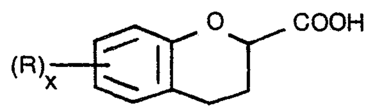

(II)

to the corresponding chroman-2-carboxamide of the following formula (III):

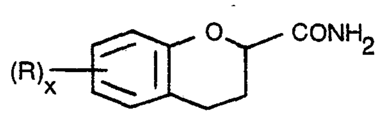

(III)

and reacting the said carboxamide (III) with a dehydrating agent to form said chroman-2-carbonitrile of the following formula (IV):

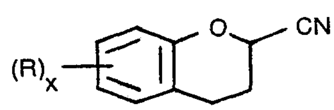

(IV)

and reacting said nitrile (IV) with ethylenediamine to form the corresponding compound of the formula (I), and wherein R and x have the meanings stated in Claim 1.

3. A process as claimed in Claim 1 which is carried out at 60°C to 120°C.

4. A process as claimed in Claim 1 or 3 which is carried out in the presence of a catalytic amount of hydrogen sulfide or carbon disulfide.

5. A process as claimed in any one of Claims 1 to 4 for the preparation of 2-(2-imidazolin-2-yl)chroman or a pharmaceutically-acceptable acid-addition salt thereof.

6. A process as claimed in any one of Claims 1 to 4 for the preparation of 2-(2-imidazolin-2-yl)-5-fluorochroman or a pharmaceutically-acceptable acid-addition salt thereof.

**Patentansprüche für die Vertragsstaaten: BE CH DE FR GB IT LI LU NL SE**

1. Chromanderivat der folgenden Formel (I):

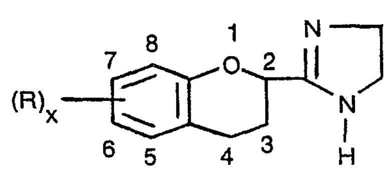

(I)

worin R für ein an die 5- und/oder 8-Stellung des Moleküls gebundenes Halogen steht und x für 0, 1 oder 2 steht; oder ein pharmazeutisch, zulässiges Säureadditionssalz davon.

2. Verbindung nach Anspruch 1, bei welcher x für 0 oder 1 steht.

3. Verbindung nach Anspruch 1 oder 2, bei welcher x für 1 steht und der Halogensubstituent R sich in der 5-Stellung befindet.

4. Verbindung nach einem der Ansprüche 1 bis 3, worin R für Fluoro steht.

5. Verbindung nach Anspruch 1, bei welcher es sich um 2 (2-Imidazolin-2-yl)chroman oder ein pharmazeutisch zulässiges Säureadditionssalz davon handelt.

6. Verbindung nach Anspruch 1, bei welcher es sich um 2-(2-Imidazolin-2-yl)-5-fluorochroman oder ein pharmazeutisch zulässiges Säureadditionssalz davon handelt.

7. Verfahren zur Herstellung einer Verbindung der folgenden Formel (I):

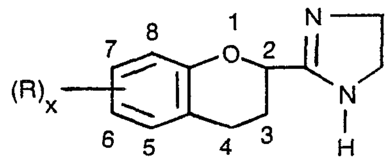

(I)

worin R und x die in Anspruch 1 angegebenen Bedeutungen besitzen, oder eines pharmazeutisch zulässigen Säureadditionssalzes davon, bei welchem eine Verbindung der folgenden Formel (IV):

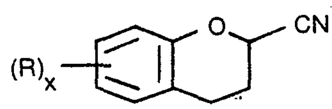

(IV)

mit Ethylendiamin umgesetzt wird.

8. Verfahren nach Anspruch 7, bei welchem eine Chroman-2-carbonsäure der folgenden Formel (II):

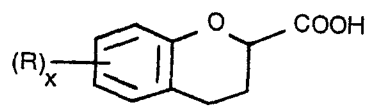

(II)

in das entsprechende Chroman-2-carboxamid der folgenden Formel (III):

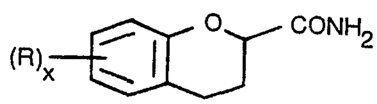

(III)

umgewandelt wird und dieses Carboxamid (III) mit einem Dehydratisierungsmittel umgesetzt wird, um das Chroman-2-carbonitril der folgenden Formel (IV):

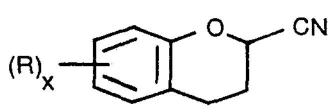

(IV)

herzustellen, und dieses Nitril (IV) mit Ethylendiamin umgesetzt wird, um die entsprechende Verbindung der Formel (I) herzustellen, worin R und x die in Anspruch 1 angegebenen Bedeutungen besitzen.

9. Pharmazeutische Zusammensetzung, welche mindestens eine Verbindung der Formel (I) von Anspruch 1, worin R und x die in Anspruch 1 angegebenen Bedeutungen besitzen, oder ein pharmazeutisch zulässiges Säureadditionssalz davon und ein pharmazeutisch zulässiges Verdünnungs-oder Trägermittel enthält.

10. Verbindung der folgenden Formel (IV):

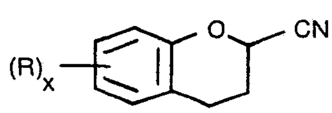

(IV)

worin R für ein an die 5- und/oder 8-Stellung des Moleküls gebundenes Halogen steht und x für 0, 1 oder 2 steht.

**Patentansprüche für den Vertragsstaat: AT**

1. Verfahren zur Herstellung einer Verbindung der folgenden Formel (I):

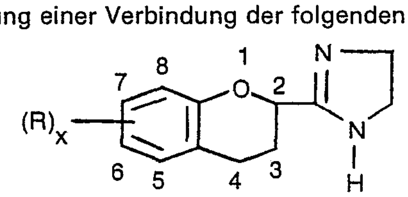

(I)

worin R für ein an die 5- und/oder 8-Stellung des Moleküls gebundenes Halogen steht und x für 0, 1 oder 2

steht, oder eines pharmazeutisch zulässigen Säureadditionssalzes davon, bei welchem eine Verbindung der folgenden Formel (IV):

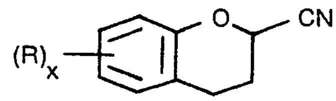

(IV)

mit Ethylendiamin umgesetzt wird.

2. Verfahren nach Anspruch 1, bei welchem eine Chroman-2-carbonsäure der folgenden Formel (II):

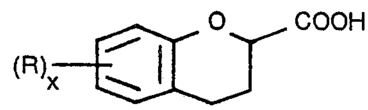

(II)

in das entsprechende Chroman-2-carboxamid der folgenden Formel (III):

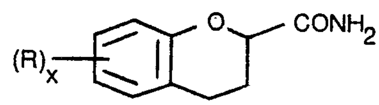

(III)

umgewandelt wird und dieses Carboxamid (III) mit einem Dehydratisierungsmittel umgesetzt wird, um das Chroman-2-carbonitril der folgenden Formel (IV);

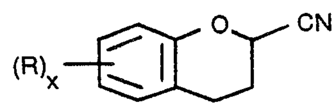

(IV)

herzustellen, und dieses Nitril (IV) mit Ethylendiamin umgesetzt wird, um die entsprechende Verbindung der Formel (I) herzustellen, worin R und x die in Anspruch 1 angegebenen Bedeutungen besitzen.

3. Verfahren nach Anspruch 1, welches bei 60 bis 120°C ausgeführt wird.

4. Verfahren nach Anspruch 1 oder 3, welches in Gegenwart einer katalytischen Menge Schwefelwasserstoff oder Schwefelkohlenstoff ausgeführt wird.

5. Verfahren nach einem der Ansprüche 1 bis 4 zur Herstellung von 2-(2-Imidazolin-2-yl)chroman oder eines pharmazeutisch zulässigen Säureadditionssalzes davon.

6. Verfahren nach einem der Ansprüche 1 bis 4 zur Herstellung von 2-(2-Imidazolin-2-yl)-5-fluorochroman oder eines pharmazeutisch zulässigen Säureadditionssalzes davon.

**Revendications pour les Etats contractants: BE CH DE FR GB IT LI LU NL SE**

1. Dérivé de chromane de formule (I) suivante:

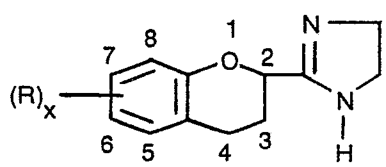

(I)

dans laquelle R est un halogène attaché à la position 5 et/ou à la position 8 de la molécule; et x a la valeur 0, 1 ou 2; ou un sel d'addition d'acide pharmaceutiquement acceptable de ce dérivé.

2. Composé suivant la revendication 1, dans lequel x a la valeur 0 ou 1.

3. Composé suivant la revendication 1 ou 2, dans lequel x a la valeur 1 et le substituant halogéno R est en position 5.

4. Composé suivant l'une quelconque des revendications 1 à 3, dans lequel R est le radical fluoro.

5. Composé suivant la revendication 1, qui est le 2-(2-imidazoline-2-yl)chromane ou un sel d'addition d'acide pharmaceutiquement acceptable de ce composé.

6. Composé suivant la revendication 1, qui est le 2-(2-imidazoline-2-yl)-5-fluorochromane ou un sel d'addition d'acide pharmaceutiquement acceptable de ce composé.

7. Procédé de préparation d'un composé de formule (I) suivante:

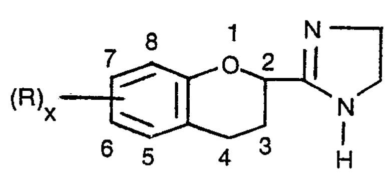

(I)

dans laquelle R et x ont les définitions indiquées dans la revendication 1, ou d'un sel d'addition d'acide pharmaceutiquement acceptable de ce composé, qui consiste à faire réagir un composé de formule (IV) suivante:

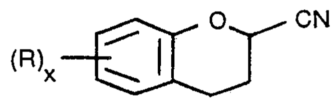
(IV)

avec l'éthylènediamine.

8. Procédé suivant la revendication 7, qui consiste à transformer un acide chromane-2-carboxylique de formule (II) suivante:

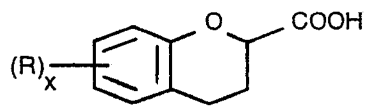
(II)

en le chromane-2-carboxamide correspondant de formule (III) suivante:

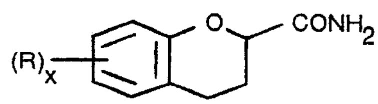
(III)

et à faire réagir ledit carboxyamide (III) avec un agent déshydratant pour former le chromane-2-carbonitrile de formule (IV) suivante:

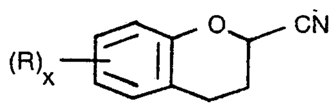
(IV)

et à faire réagir le nitrile (IV) avec l'éthylènediamine pour former le composé correspondant de formule (I), R et x ayant les définitions indiquées dans la revendication 1.

9. Composition pharmaceutique comprenant au moins un composé de formule (I) suivant la revendication 1, dans laquelle R et x ont les définitions indiquées dans la revendication 1, ou un sel d'addition d'acide pharmaceutiquement acceptable de ce composé et un diluant ou support acceptable du point de vue pharmaceutique.

10. Composé de formule (IV) suivante:

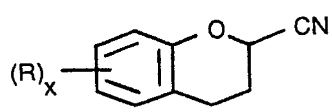
(IV)

dans laquelle R est un halogène attaché à la position 5 et/ou à la position 8 de la molécule; et x a la valeur 0, 1 ou 2.

**Revendications pour l'Etat contractant: AT**

1. Procédé de préparation d'un composé de formule (I) suivante:

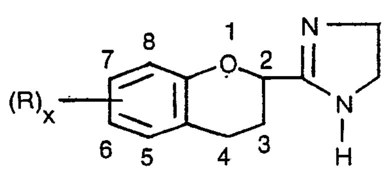
(I)

dans laquelle R est un halogène attaché à la position 5 et/ou à la position 8 de la molécule et x à la valeur 0, 1 ou 2, ou d'un sel d'addition d'acide pharmaceutiquement acceptable de ce composé, qui consiste à faire réagir un composé de formule (IV) suivante:

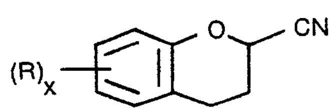
(IV)

avec l'éthylènediamine.

2. Procédé suivant la revendication 1, qui consiste à transformer un acide chromane-2-carboxylique de formule (II) suivante:

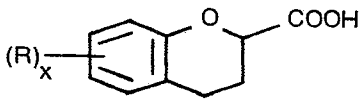

(II)

en le chromane-2-carboxamide correspondant de formule (III) suivante:

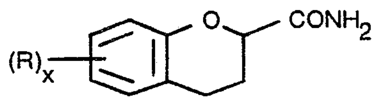

(III)

et à faire réagir ledit carboxamide (III) avec un agent déshydratant pour former le chromane-2-carbonitrile de formule (IV) suivante:

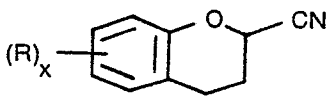

(IV)

et à faire réagir le nitrile (IV) avec l'éthylènediamine pour former le composé correspondant de formule (I), R et x ayant les définitions données dans la revendication 1.

3. Procédé suivant la revendication 1, qui est mis en oeuvre à une température de 60 à 120°C.

4. Procédé suivant la revendication 1 ou 3, qui est mis en oeuvre en présence d'un quantité catalytique de sulfure d'hydrogène ou de sulfure de carbone.

5. Procédé suivant l'une quelconque des revendications 1 à 4 pour la préparation du 2-(2-imidazoline-2-yl)chromane ou d'un sel d'addition d'acide pharmaceutiquement acceptable de ce composé.

6. Procédé suivant l'une quelconque des revendications 1 à 4 pour la préparation du 2-(2-imidazoline-2-yl)-5-fluorochromane ou d'un sel d'addition d'acide pharmaceutiquement acceptable de ce composé.